# EUROPÄISCHE PATENTSCHRIFT

(11) **EP 3 432 941 B1**
(45) Veröffentlichungstag und Bekanntmachung des Hinweises auf die Patenterteilung: **01.07.2020**
(21) Anmeldenummer: 17711145.7
(22) Anmeldetag: 15.03.2017
(51) Int. Cl.: A61L 27/30, A61L 27/54, A61L 27/56

(54) **IMPLANTATKÖRPER MIT WIRKSTOFFTRÄGERSCHICHT UND ABGABEKONTROLLSCHICHT**
IMPLANT BODY WITH ACTIVE SUBSTANCE CARRIER LAYER AND RELEASE CONTROL LAYER
CORPS D'IMPLANT COMPORTANT UNE COUCHE PORTEUSE D'AGENT ACTIF ET UNE COUCHE DE COMMANDE DE LIBÉRATION

(30) Priorität: 23.03.2016 DE 102016204899
(43) Veröffentlichungstag der Anmeldung: 30.01.2019
(73) Patentinhaber: Fraunhofer Gesellschaft zur Förderung der angewandten Forschung e.V., 80686 München (DE)
(72) Erfinder: BORCHERDING, Kai, 28865 Lilienthal (DE); SPECHT, Uwe, 28209 Bremen (DE); GRUNWALD, Ingo, 28865 Lilienthal (DE); SALZ, Dirk, 28195 Bremen (DE); IHDE, Jörg, 28865 Lilienthal (DE); LUKASCZYK, Thomas, 27721 Ritterhude (DE)
(74) Vertreter: Eisenführ Speiser
(86) Internationale Anmeldenummer: PCT/EP2017/056167
(87) Internationale Veröffentlichungsnummer: WO 2017/162502

(56) Entgegenhaltungen:
- US-A1- 2009 118 821

## Beschreibung

Die Erfindung betrifft einen Implantatkörper mit einer Wirkstoffträgerschicht und einer Abgabekontrollschicht sowie die Verwendung einer Abgabekontrollschicht zur Verringerung des Porenöffnungsdurchmessers von Poren auf einem Implantatkörper und ein Verfahren zum Erzeugen eines entsprechenden Implantatkörpers.

Beim Implantieren eines Implantates in einen Körper kann es aus vielerlei Gründen zu Problemen kommen: zum einen kann die Akzeptanz des Implantates durch den Körper ein Problem sein (Abstoßungsreaktion), zum anderen können beispielsweise Anwachsbeziehungsweise Einwachsprobleme vorliegen. Darüber hinaus besteht die Gefahr, dass über die Implantate oder während des Implantationsprozesses Keime in den Empfängerkörper gelangen. Nosokomiale Infektionen und der Ausfall von Implataten wie z.B. Endoprothesen führen zu einem hohen Mehraufwand in der Gesundheitsversorgung und somit zu deutlich erhöhten Kosten. Im Extremfall kann eine Infektion mit einem multiresistenten Keim zur Re-Implantation oder sogar zum Tod des Patienten führen.

Bisherige Ansätze zur Verhinderung von Infektionen, die durch bakteriell belastete Implantate verursacht werden, finden in der Praxis keine Anwendung, da diese keine lang anhaltende Freisetzung der antimikrobiell wirkenden Substanzen gewährleisten können bzw. keine Stabilität gegen übliche Sterilisationsverfahren aufweisen. Ferner wird bei den genutzten Operationstechniken die aktive Substanz inhomogen verteilt, so dass keine gleichmäßige Wirkung erreicht wird.

Bei der systemischen Gabe von Wirkstoffen, wie zum Beispiel Antibiotika, ist heutzutage immer noch umstritten, wie das Antibiotikum in der richtigen Dosis in das Implantationsfeld gelangt. Des Weiteren ist die systemische Antibiose in wenig durchbluteten Körperteilen, wie zum Beispiel im Knochen, erschwert, wie am Beispiel des wenig durchbluteten Grenzbereichs zwischen Knochen und Implantat vorzufinden.

Ferner benötigen Medizinprodukte, wie zum Beispiel Traumaimplantate, eine lange Haltbarkeit nach Sterilisation. Übliche Sterilisationsmethoden (zum Beispiel Gammasterilisation) können Wirkstoffe verändern.

Zum Nachweis, dass sich der Wirkstoff durch die Sterilisation nicht verändert, und immer noch die gleiche Wirkung entfaltet, sind regulatorisch hohe Studienkosten bei der Entwicklung einzukalkulieren. Im Stand der Technik ist die Ausstattung von Implantaten mit einer silberhaltigen Beschichtung bekannt. Das Silber hat hierbei antibakterielle Wirkung. Die DE 10353756 offenbart die Einbettung von Silbernanopartikeln in einer silikatischen Matrix. Problematisch bei diesem Verfahren ist, dass nur metallische Wirkstoffe, wie zum Beispiel Silber, eingesetzt werden können, da durch die für die Schichterzeugung notwendige Plasmatechnologie ein in den Schichten verankerter Wirkstoff zerstört werden würde.

Die DE 10225324 offenbart einen antibakteriellen Lack, der metallisches Silber in Form von Partikeln beinhaltet. Aufgrund der hohen Aushärtungstemperaturen, beziehungsweise der Anwendung von UV-Strahlung, können auch in diesem Fall keine empfindlichen Wirkstoffe wie herkömmliche Antibiotika verarbeitet werden.

Als alternative Technologie können Wirkstoffe wie Antibiotika auf Oberflächen fixiert werden, in dem diese mit einer Fettsäure modifiziert werden. Dieser Ansatz ermöglicht zwar eine langanhaltende Freisetzung der Antibiotika, jedoch ist die mechanische Abriebfestigkeit der Beschichtung gering. Zudem müssten entsprechende Implantate ein langwieriges Zulassungsverfahren durchlaufen.

US 2009/0118821 A1 offenbart eine Endoprothese mit einem porösen Reservoir für einen Wirkstoff, um die Elution des Wirkstoffs aus dem Reservoir zu steuern.

Die DE 10 227938 A1 offenbart Antibiotika enthaltende Beschichtungen, wobei die Wirkstoffe mithilfe eines polymeren Schichtbildners fixiert werden. Nachteilig an diesem Verfahren ist, dass die Sterilisation des Implantates nur mit Hilfe von Ethylenoxid erfolgen kann. Eine derartige Behandlung kann jedoch zu Rückständen oder Reaktionsprodukten auf der Implantatoberfläche führen, in der medizinisch Unbedenklichkeit nicht gewährleistet ist.

DE 10 2008 001 014 A1 offenbart ein antimikrobielles und nicht zytotoxisches Schichtmaterial sowie Verwendungen dieses Schichtmaterials.

Vor diesem Hintergrund war es Aufgabe der vorliegenden Erfindung, Implantate zu entwickeln, die mit einer Vielzahl von Wirkstoffen beladen werden können (in Kombination oder alleine), wobei bevorzugt die Beladung auch kurz vor der Implantation durchgeführt werden kann. Gleichzeitig sollten die Implantate in der Lage sein, Wirkstoffe über einen langen Zeitraum freizusetzen, sodass diese lokal ihre Wirkung entfalten können. Es war auch bevorzugt Aufgabe der vorliegenden Erfindung, langfristige Zulassungsverfahren für die Zulassung eines pharmazeutischen Produktes zu vermeiden.

Die erfindungsgemäße Aufgabe wird gelöst durch einen Implantatkörper, umfassend ein Substrat und auf dessen Oberfläche ein Schichtsystem aus einer Wirkstoffträgerschicht und darauf angeordnet eine Abgabekontrollschicht, wobei die Wirkstoffträgerschicht eine Vielzahl von offenen Poren umfasst und die Abgabekontrollschicht eine aus der Gasphase abgeschiedene Schicht ist, die die Fläche der Porenöffnung der Poren der Wirkstoffträgerschicht reduziert, ohne sie zu schließen.

Überraschenderweise hat sich herausgestellt, dass sich durch die Kombination einer aus der Gasphase abgeschiedenen Abgabekontrollschicht mit einer offenporigen Wirkstoffträgerschicht geeignete Systeme zur Lösung der oben beschriebenen Aufgabe erstellen lassen.

Im Sinne der vorliegenden Erfindung ist ein "Wirkstoff" ein Stoff, der nach der Implantation des Implantatkörpers in den Empfängerkörper aus der Wirkstoffträgerschicht austreten und im Körper eine positive Wirkung erzeugen kann. Bevorzugte Wirkstoffe im Sinne der vorliegenden Erfindung sind medizinische Wirkstoffe, das heißt Wirkstoffe mit einer arzneilichen Wirkung, oder Wirkstoffe, die die Akzeptanz des Implantates im Empfängerkörper verbessern. Letztere können zum Beispiel Wirkstoffe sein, die das Einwachsverhalten verbessern und/oder Abstoßungsreaktionen verringern oder vermeiden. Bevorzugte medizinische Wirkstoffe sind antimikrobielle Wirkstoffe oder Chemotherapeutika, besonders bevorzugt ist als medizinischer Wirkstoff ein Antibiotikum, ein antimikrobielles Peptid, ein Antiseptikum, Knochenstoffwechsel beeinflussende Wirkstoffe (insbesondere Knochenmorphogenetische Proteine (BMP)), Hyaluronsäure oder ein Phytoalexin.

"Offene Poren" im Sinne der vorliegenden Erfindung sind solche Poren, die einen Flüssigkeits- oder Gasaustausch aus dem Poreninneren mit der Umgebung des Implantatkörpers ermöglichen.

Eine "Reduktion der Porenöffnung, ohne die Poren zu schließen", liegt im Sinne der vorliegenden Anmeldung dann vor, wenn der Querschnitt der Porenöffnung vor der Reduktion eine größere Fläche aufweist als nach der Reduktion, wobei aber eine Porenfläche verbleibt, die noch immer einen Gas-/Flüssigkeitsaustausch des Poreninneren mit seiner Umgebung (außerhalb des Implantatkörpers) ermöglicht.

Im Sinne der vorliegenden Erfindung ist im Zweifelsfall die Größe der Porenöffnung vor Reduktion an der Grenzfläche zwischen Wirkstoffträgerschicht und Abgabekontrollschicht zu messen, die Porenöffnung nach Flächenreduktion auf der Oberfläche der Abgabekontrollschicht, die vom Implantatkörper weggerichtet ist.

Eine _{"}Reduktion der Porenöffnung der Poren, ohne sie zu schließen", bedeutet bevorzugt im Sinne der vorliegenden Erfindung, dass die Reduktion (durch die Abgabekontrollschicht) so erfolgt, dass ≥ 50%, bevorzugt ≥ 60 %, bevorzugt ≥ 70 %, weiter bevorzugt ≥ 80 %, noch weiter bevorzugt ≥ 90 %, und besonders bevorzugt ≥ 95 % der Poren der Wirkstoffträgerschicht, die vor Abscheiden der Abgabekontrollschicht Poren waren, unverschlossen verbleibt.

Bevorzugt im Sinne der vorliegenden Erfindung ist, dass das Substrat des Implantatkörpers aus Titan, Edelstahl, Magnesium, Niob, Tantal und deren Legierungen, Polymeren (insbesondere PEEK, PE), Keramiken und/oder Kompositmaterialien besteht.

Bevorzugt im Sinne der vorliegenden Erfindung ist ein erfindungsgemäßer Implantatkörper, wobei die Wirkstoffträgerschicht eine metallhaltige oder metalloxidhaltige Schicht ist oder aus Metall oder Metalloxid besteht und es sich um Metalle oder Metalloxide der Elemente Ti, Fe, Cr, Co, V oder Ta handelt.

Im Sinne der Erfindung ist es möglich, dass die Wirkstoffträgerschicht auch eine Legierung, insbesondere der vorgenannten bevorzugten Elemente ist oder eine Mischung aus den Metalloxiden der vorgenannten bevorzugten Elemente.

Bevorzugt im Sinne der vorliegenden Erfindung ist es, dass das Substrat des erfindungsgemäßen Implantatkörpers ein Metall ist und die Wirkstoffträgerschicht aus Oxiden des das Substrat bildenden Metalls besteht oder im Wesentlichen besteht.

Der Vorteil in dieser Kombination ist darin zu sehen, dass an der Oberfläche des Substrates durch geeignete Behandlung Poren und damit eine Wirkstoffträgerschicht im Sinne der vorliegenden Erfindung erzeugt werden können.

Bevorzugte Verfahren zur Erzeugung der im erfindungsgemäßen Implantatkörper vorhandenen Wirkstoffträgerschicht sind Verfahren, ausgewählt aus der Gruppe bestehend aus Laserstrukturierung, laserinduzierte physikalische Gasphasenabscheidung (Laser-PVD), Laserstrahlverdampfen (PLD), laserinduzierte chemische Gasphasenabscheidung (Laser-CVD), Micro-Arc Oxidation (MAO), elektrochemische Oxidation und Anodisierung.

Die genannten Verfahren haben sich besonders geeignet erwiesen, um die erfindungsgemäß vorzusehende Wirkstoffträgerschicht zu erzeugen.

Die Wirkstoffträgerschicht definiert sich dabei bevorzugt durch den Bereich abweichender chemischer Zusammensetzung verglichen mit dem Substrat. Grundsätzlich ist es jedoch auch möglich, dass die Wirkstoffträgerschicht die gleiche Zusammensetzung hat wie das das Substrat, wenn man von den Poren absieht. Dies kann zum Beispiel dann der Fall sein, wenn die Wirkstoffträgerschicht aus einem metallischen Substrat, insbesondere durch eines der oben als bevorzugt beschriebenen Verfahren, erzeugt wird, unter Bedingungen, die eine Oxidation ausschließen. So ist es zum Beispiel möglich, metallische Substrate unter einer Schutzgasatmosphäre mit der Wirkstoffträgerschicht zu versehen.

In Fällen, in denen sich die Wirkstoffträgerschicht - mit Ausnahme der Poren - nicht stofflich von der Substratzusammensetzung unterscheidet, soll für den Zweck dieses Textes die Wirkstoffträgerschicht der Bereich der Substratschicht sein, in dem 95 Prozent aller Poren vollständig enthalten sind. Dies bedeutet, dass die übrigen 5 Prozent der Poren noch tiefer in das Substrat hineinragen können. Dabei ist in einem solchen Fall die Wirkstoffträgerschicht die gesamte poröse Oberfläche des Substrates mal der Tiefe, in der die genannte 95 Prozent der Poren vollständig enthalten sind.

Bevorzugt ist ein erfindungsgemäßer Implantatkörper, bei dem die Abgabekontrollschicht eine CVD-, PE-CVD- oder PVD-Schicht ist. Eine CVD-, PE-CVD- oder PVD-Schicht im Sinne dieser Erfindung ist eine aus der Gasphase bei Atmosphärendruck oder im Niederdruck abgeschiedene Beschichtung. Eine genaue Beschreibung und Unterscheidung dieser Beschichtungsverfahren findet sich z.B. unter Milton Ohring, "The Materials Science of Thin Films", Academic Press, San Diego New York Boston, 1990 ISBN 0-12-524990-X.

Bevorzugt im Sinne der vorliegenden Erfindung ist ein Implantatkörper, bei dem die Abgabekontrollschicht zu ≥ 90% aus Ti, C, O und/oder Si besteht, bezogen auf die in der Schicht enthaltenen Atome ohne H gemessen mittels XPS.

Weiterhin ist es bevorzugt, dass die Abgabekontrollschicht bevorzugt eine (plasmapolymere) siliziumorganische oder kohlenstoffhaltige Titandioxid-Beschichtung ist. Die Zusammensetzung der kohlenstoffhaltigen Titandioxidbeschichtung entspricht bevorzugt

| | |
|---|---|
| O: | 55,0 at% - 75 at% |
| Ti: | 20 at% - 44,5 at% |
| C: | 0,05 - 15 at%, |

jeweils gemessen mittels XPS, bezogen auf die in der Schicht enthaltenen Atome ohne H.

Die Zusammensetzung der siliziumorganischen Beschichtung entspricht bevorzugt

| | |
|---|---|
| O: | 40,0 at% - 60,0 at% |
| Si: | 25,0 at% - 33,0 at% |
| C: | 3,0 - 30,0 at%, |

ebenfalls jeweils gemessen mittels XPS, bezogen auf die in der Schicht enthaltenen Atome ohne H.

Bevorzugt sind die Beschichtungen, die einen Kohlenstoffanteil von 3 at% oder weniger enthalten. Die stöchiometrische Zusammensetzung der Oxide ist daher SiOx und TiOx mit einem kleinen Anteil von Kohlenstoff.

Ferner ist bevorzugt, dass die Abgabekontrollschicht biokompatibel nach DIN EN ISO 10993-5 (Biologische Beurteilung von Medizinprodukten - Teil 5: Prüfungen auf In-vitro-Zytotoxizität) ist.

Insbesondere für die Bestimmung der Zusammensetzung der Wirkstoffträgerschicht ist zu berücksichtigen, dass die Füllung der Poren der Wirkstoffträgerschicht nicht zu der Wirkstoffträgerschicht selbst gehört, sofern in diesem Text von Wirkstoffträgerschicht die Rede ist.

Bevorzugt ist ein erfindungsgemäßer Implantatkörper, wobei die Poren wenigstens teilweise mit einem medizinischen Wirkstoff, bevorzugt einem antimikrobiellen Wirkstoff oder einem Chemotherapeutikum und besonders bevorzugt einem Antibiotikum gefüllt sind.

Erfindungsgemäß bevorzugt ist es, dass der (medizinische) Wirkstoff in Form einer wässrigen Lösung vorliegt.

Weiterhin ist es erfindungsgemäß bevorzugt, dass die Abgabekontrollschicht zumindest nach außen hin hydrophil ist. Hydrophil im Sinne der vorliegenden Erfindung bedeutet, dass sie einen statischen Wasserrandwinkel gemessen nach DIN 55660-2 von ≤ 90 °, bevorzugt ≤ 75°, weiter bevorzugt ≤ 50° aufweist.

Bevorzugt ist ein erfindungsgemäßer Implantatkörper, wobei der Porendurchmesser 0,005 µm bis 2,0 µm vor Aufbringen der Abgabekontrollschicht, bevorzugt 0,1 µm bis 1,5 µm, besonders bevorzugt 0,5 µm gemessen mittels Rasterelektronenmikroskopie beträgt.

Der "Porendurchmesser" im Sinne der vorliegenden Erfindung ist der durchschnittliche Durchmesser der einzelnen Pore im Bereich der Wirkstoffträgerschicht. Dieser wird einer rasterelektronischen Aufnahme der Oberfläche bestimmt.

Bevorzugt im Sinne der vorliegenden Erfindung ist, dass die Wirkstoffträgerschicht über eine Porosität von ≥ 15 Prozent, bevorzugt 18 bis 25 Prozent und besonders bevorzugt 20 bis 30 Prozent verfügt.

Unter der "Porosität" im Sinne der vorliegenden Erfindung ist hierbei der Porenvolumenanteil am Volumen der Wirkstoffträgerschicht zu verstehen. Bestimmt kann dies werden durch die Massenzunahme der porösen Schicht nach der Füllung der Poren mit Wasser.

Hierfür erfolgt eine Wägung der Substratoberfläche vor der Porenbeladung mit einer ausreichend feinen Waage. Die Probe wird im Anschluss zur Imprägnierung in Wasser eingelegt. Die Dauer der Imprägnierung sollte mindestens 24 h betragen. Nach dem Abtrocknen der Oberfläche erfolgt eine erneute Wägung der Probe. Mit Hilfe der Masse-änderung Δm lässt sich das Porenvolumen V errechnen nach V_{H2O} = Δm / δ, wobei δ die Dichte des Wassers ist. Die Porosität der Schicht ergibt sich dann durch das Verhältnis zwischen dem Porenvolumen V = V_{H2O} und dem Gesamtvolumen der Beschichtung.

Ferner ist im Sinne der vorliegenden Erfindung bevorzugt, dass der Porendurchmesser bezogen auf den kleinsten Durchmesser der Pore innerhalb der Wirkstoffträgerschicht im Zahlenmittel 0,01 bis 1,8 µm, bevorzugt 0,1 bis 1,2 µm beträgt und/oder dass die Massenaufnahme pro Quadratzentimeter Oberfläche mindestens 200 µg beträgt.

Porendurchmesser werden im Zweifelsfall mittels Rasterelektronenmikroskopie in Aufsicht gemessen.

Bevorzugt im Sinne der vorliegenden Erfindung ist ein erfindungsgemäßer Implantatkörper, wobei der Porenöffnungsdurchmesser 0,002 µm bis 1,0 µm nach Aufbringen der Abgabekontrollschicht, bevorzugt 0,05 µm bis 0,5 µm, besonders bevorzugt 0,1 µm gemessen mittels Rasterelektronenmikroskopie beträgt.

Sofern von "Durchmesser" ohne weiteren Hinweis in diesem Text die Rede ist, betrifft dies jeweils den größten messbaren Durchmesser bei nicht-kreisförmigen (z.B. ovalen) Flächen.

Der Porenöffnungsdurchmesser im Sinne der vorliegenden Erfindung ist der Durchmesser, den die Pore nach außen hin also an der Außenseite der Abgabekontrollschicht besitzt. Auch der Porenöffnungsdurchmesser wird im Zweifelsfall mittels Rasterelektronenmikroskopie gemessen.

Bevorzugt ist, dass das Zahlenmittel des Porenöffnungsdurchmessers 0,005 µm bis 0,75 µm, besonders bevorzugt 0,1 µm bis 0,5 µm gemessen mittels Rasterelektronenmikroskopie beträgt.

Der erfindungsgemäße Implantatkörper ist aufgrund der erfindungsgemäßen einzusetzenden Beschichtung in der Lage, lokal im Implantationsfeld den Wirkstoff freizusetzen. Dies gilt insbesondere für die oben beschriebenen bevorzugten Ausführungsvarianten.

Dabei ist es dem Fachmann möglich, durch angemessene Variation der Porengrößen und der Porosität in der Wirkstoffträgerschicht in Kombination mit der durch die Abgabekontrollschicht gewährleistete Porenöffnungsgröße eine langanhaltende Freisetzung des medizinischen Wirkstoffes direkt an dem Ort zu gewährsleisten, wo der Wirkstoff benötigt wird. Somit wird zum Beispiel der antimikrobielle Wirkstoff, wie zum Beispiel ein Antibiotikum, gezielt in den infektionsrelevanten Bereich gebracht und somit das Risiko einer postoperativen oder nosokominalen Infektion gesenkt.

Der erfindungsgemäße Implantatkörper, insbesondere in seinen bevorzugten Ausführungsformen, ermöglicht es, dass das Implantat mittelbar oder unmittelbar vor der Implantation des Implantates mit einem oder mehreren Wirkstoffen, wie zum Beispiel einem Antibiotikum, infiltriert bzw. beladen werden kann. Dies kann zum Beispiel dadurch passieren, dass das Implantat in eine sterile Wirkstofflösung unmittelbar vor oder sogar während der Operation eingelegt wird. Somit sind erprobte Wirkstofflösungen einsetzbar, und es entfällt die Notwendigkeit, dass nachgewiesen werden muss, dass der Wirkstoff noch nach einer sonst üblichen Sterilisation seine Wirksamkeit behält. Dadurch werden hohe Kosten für die Zulassung eines pharmazeutischen Produktes als Kombination von Implantat und Wirkstoff vermieden.

Die Infiltration/Beladung des erfindungsgemäßen Implantatkörpers kann durch Einstellung der Oberflächenenergie der Abgabekontrollschicht erleichtert werden: Sofern beispielsweise die Infiltration/Beladung mittels einer wässrigen Lösung erfolgen soll, wird der Fachmann die Oberflächenenergie der Abgabekotrollschicht so einstellen, dass diese hydrophil wirkt. Auch die Geometrie der Poren wie zum Beispiel die Porengröße, das Porenvolumen, der Porendurchmesser und insbesondere auch der Porenöffnungsdurchmesser im Bereich der Abgabekontrollschicht können auf die speziellen Anforderungen eines Wirkstoffes wie zum Beispiel an dessen Viskosität und auch die Oberflächenenergie angepasst werden. Sofern der Fachmann das Freigabeintervall / Freigabekinetik des oder der Wirkstoffe vergrößern bzw. verkleinern möchte, so stehen ihm eine Reihe von Maßnahmen zur Verfügung:
- Verringerung des Porenöffnungsdurchmessers im Rahmen der Abgabekontrollschicht,
- Verringerung des kapillaren Durchmessers der Poren,
- nach außen hin verjüngende Struktur der Poren,
- Volumengröße der Poren,
- Ausnutzen von Quellvorgängen von Wirkstoff und/oder Wirkstoffträgerschicht, Wirkstoffträgerschicht bzw. einer Wirkstoffträgersubstanz (z.B. Hydrogel),
- kristallografische Umwandlungen von Wirkstoff/Schichtsystem,
- Vorsehen einer weiteren, zusätzlichen biokompatiblen und biodegradierbaren Beschichtung, beispielsweise auch einer geschlossenen Beschichtung, die sich erst im Körper löst, um die darunterliegenden Porenöffnungen freizugeben,
- Erhöhung der Schichtdicke bei konstanter Porosität
- Aufbringen einer Beschichtung, die stimuliresponsiv ist, wie z.B. durch pH, Licht, Energieveränderung (z.B. Wärme/ Kälte), und hierdurch eine Freisetzung erlaubt
- Aufbringen einer Kontrollschicht mit Porenöffnungen, die in Relation zu den Porenöffnungen der Wirkstoffträgerschicht lateral versetzt sind; hierdurch entsteht eine Verkleinerung der effektiven Porenöffnung der Wirkstoffträgerschicht
- Aufbringen einer Kontrollschicht mit vertikal ausgeprägten Poren, wodurch der Diffusionsweg des Wirkstoffs durch die Poren deutlich verlängert wird; die Poren in der Kontrollschicht müssen dabei nicht unbedingt zu einer Verkleinerung des Porenöffnungsdurchmessers im Vergleich zur reinen Wirkstoffschicht führen

Grundsätzlich ist es auch möglich, bewusst in die Wirkstoffträgerschicht verschiedene Porengrößen einzubringen, um so sich überlagernde Freigabeprofile für den Wirkstoff (oder auch für verschiedene Wirkstoffe) in Abhängigkeit von der jeweiligen Porengröße bzw. Porengeometrie zu gewährleisten. So wäre es beispielweise möglich, verschiedene Freigabepeaks zu erzeugen.

Ein weiterer Vorteil des erfindungsgemäßen Implantatkörpers liegt darin, dass das Implantat vor der Beladung mit dem Wirkstoff beispielsweise mittels Gammastrahlen oder anderen potenziell den wirkstoffzerstörenden Sterilisationsmethoden sterilisiert werden kann. Unter den geeigneten Bedingungen kann dann ein entsprechendes Implantat kurz vor oder während der Operation mit dem Wirkstoff beladen werden, ohne dass dieser durch die Sterilisationsmethode an Wirkung verliert.

Darüber hinaus kann die Wirkstoffwirkung des erfindungsgemäßen Implantates mit anderen Methoden, wie zum Beispiel der systemischen Gabe, kombiniert werden.

Im Sinne der Erfindung ist es in diesem Zusammenhang bevorzugt, dass die Abgabekontrollschicht und/oder die Wirkstoffträgerschicht Silber und/oder Kupfer umfasst. Auf diese Art besteht ein zusätzlicher antimikrobieller Effekt, der die Infektionswahrscheinlichkeit im Zusammenhang mit dem erfindungsgemäßen Implantatkörper nochmals verringert.

Teil der Erfindung ist auch die Verwendung einer Abgabekontrollschicht wie oben definiert, zur Verringerung des Porenöffnungsdurchmessers der Poren auf einem Implantatkörper, wobei die Pore nicht verschlossen wird und bevorzugt mit einem medizinischen Wirkstoff wenigstens teilweise befüllt ist.

Durch den Einsatz der erfindungsgemäß einzusetzenden Abgabekontrollschicht werden die oben beschriebenen positiven Effekte ermöglicht.

Teil der Erfindung ist ferner ein Verfahren zum Erzeugen eines erfindungsgemäßen Implantatkörpers, umfassend die Schritte
a) Bereitstellen eines Substrates für den Implantatkörper,
b) Erzeugen einer porenhaltigen Wirkstoffträgerschicht auf der Oberfläche des Implantatkörpers und
c) Abscheiden einer Abgabekontrollschicht aus der Gasphase auf die Wirkstoffträgerschicht, so dass der Porendurchmesser der Poren der Wirkstoffträgerschicht verringert wird, ohne dass die Poren verschlossen werden.

Dabei sind die jeweilig oben als bevorzugt beschriebenen Materialien für die einzelnen Schritten selbstverständlich ebenfalls bevorzugt.

Bevorzugt ist ein erfindungsgemäßes Verfahren, bei dem die Beladung des Implantatkörpers mit dem medizinischen Wirkstoff erst ≤ 3 Stunden vor Implantation, bevorzugt ≤ 2 Stunden vor Implantation, weiter bevorzugt ≤ 1 Stunde vor Implantation, noch weiter bevorzugt ≤ 0,5 Stunden vor der Implantation erfolgt.

Insbesondere ist es möglich, und auch im Sinne der Erfindung häufig bevorzugt, dass die Beladung erst unmittelbar vor Implantation des Implantates abgeschlossen wird.

Nachfolgend wird die Erfindung anhand der Figuren und der Beispiele weiter erläutert.

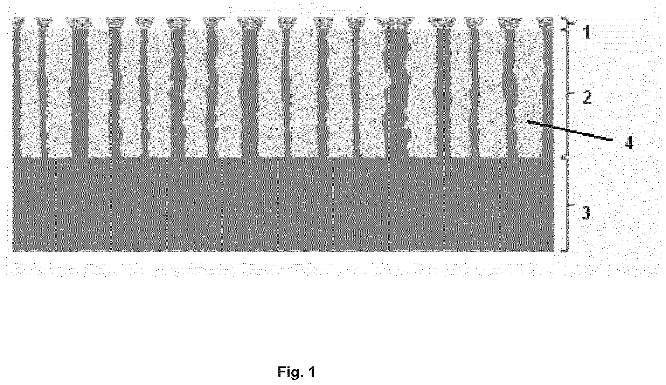

Die Figur 1 ist dabei ein schematischer Ausschnitt eines erfindungsgemäßen Implantatkörpers, wobei die Bezugszeichen Folgendes bedeuten:
1 Abgabekontrollschicht
2 Wirkstoffträgerschicht
3 Substrat
4 Pore, gegebenenfalls mit Wirkstoff gefüllt

Figur 2 stellt das Zeit-Konzentrationsverhalten der Freigabe von Silberionen in Ringer-Lösung für einen beispielhaften mit einer silberhaltigen Beschichtung versehenen erfindungsgemäßen Implantatkörper dar.

Figur 3 stellt den zeitlichen Verlauf der Freigabe von Methylenblau aus einer mit Wirkstoff beladenen Titandioxidschicht als Wirkstoffträgerschicht in einem erfindungsgemäßen Implantatkörper dar.

Figur 4 zeigt rasterelektronische Aufnahmen der Oberfläche der MAO-Schicht vor (links) und nach (rechts) der Applikation mit der Verjüngungsschicht.

### Beispiele

### Beispiel 1:

Ein flaches Titanimplantat (Grade 2) wird mit einem gepulsten Nd:YAG-Laser der Wellenlänge 1064 nm (CL250; Firma CleanLaser) strukturiert. Die Behandlung erfolgt dabei mit einer mittleren Leistung von 205 Watt, 12 kHz, 100 ns Pulslänge, 0,25 ms On-Time pro Fokusfläche bzw. 12 Repetitionen auf einer Fokusfläche von 680 µm - Durchmesser (FWHM, Top-Hat-Profil), und einer 2D-Scan-Optik bei einem Fokusflächenrasterabstand in x- und y-Richtung von 0,05 mm, in einem Zyklus. Die so erzeugte Struktur besteht aus Titandioxid und weist eine Dicke von ca. 2 µm auf. Die Porentiefe beträgt ca. 200 nm und der Porendurchmesser 10 nm.

Die Poren werden durch ein CVD-Verfahren mit Titandioxid als Abgabekontrollschicht verjüngt (Durchführung siehe unten). Hierzu wird ein gasförmiger, Titan-haltiger und hydrolyseempfindlicher Precursor und Wasser als Reaktand auf die Oberfläche des strukturierten Implantats geleitet. Alternativ kann auch die Restfeuchte in der umgebenden Atmosphäre als zweiter Reaktand dienen. Durch die Hydrolyse von Titantetraisopropoxid (TTIP) scheidet sich auf der Oberfläche der Struktur eine Titandioxidschicht ab, die die ursprünglich vorhandenen Poren nicht verschließt, sondern nur den Porenöffnungsdurchmesser verringert. Nachfolgend sind die Abscheidebedingungen für die Erzeugung einer Abgabekontrollschicht auf Basis des CVD-Verfahrens beschrieben:
- Beschichtungsaufbau: Behandlung unter atmosphärischen Bedingungen ohne gekapseltes System; es wurde nur eine Einspeisung für den Titan-haltigen Precursor verwendet; als Reaktionspartner diente die Restfeuchte in der Atmosphäre (typischerweise 25%r.F. - 30%r.F.);
- Geometrie Beschichtungsdüse: kreisrunde Düse mit 4 mm Innendurchmesser
- Titan-Precursor: Titanisopropoxid
- Trägergas Titan-Precursor: Stickstoff 5.0, 5 l/min
- Menge Titan-Precursor: 10 µl/min
- Winkel Beschichtungsdüse: 0° (senkrecht zur Probe)
- Abstand Beschichtungsdüse: 30 mm
- Probenhalterung: Fixierung direkt auf dem Verfahrtisch
- Verfahrgeschwindigkeit: 7 m/min
- Zeilenabstand für mäanderförmige Probenrasterung: 1 mm
- Probentemperatur während der Beschichtung: 80 °C
- Art der Probenheizung während der Beschichtung: Erwärmung durch beheizte Unterlage (Widerstandsheizung);

### Beispiel 2:

Ein Edelstahlimplantat wird mit einem UV-Laser (COMPexPro 205F; Firma Coherent) behandelt.Die Behandlung erfolgt bei einer Wellenlänge von 248 nm mit einer Frequenz von 50 Hz, einer Pulsdauer von 25 ns, einer Energiedichte von 400 mJ/cm² und einer Wiederholrate von 1000 Pulsen/cm². Hierbei entsteht eine offenporige Struktur aus Chrom- und Eisenoxid. Die Verjüngung der Poren erfolgt mit Hilfe eines Atmosphärendruckplasmaprozesses, der neben einer SiₓO_{y}C_{z}-Schicht zusätzlich Silbernanopartikel auf der Oberfläche der Struktur abscheidet Die Einbettung der Silbernanopartikel erfolgt dabei im Rahmen eines Multilagenaufbaus bestehend aus 40 nm dicken SiₓO_{y}C_{z}-Grundschicht, Silberpartikellage und einer 40 nm dicken SiₓO_{y}C_{z}-Deckschicht. Die Abscheidung der Silberpartikel erfolgte mittels eines plasmainduzierten Sputterns bei Atmosphärendruck durch Zufuhr einer Drahtelektrode aus Silber in die relaxierende Plasmazone. Die Beschichtungsparameter für die Erzeugung der einzelnen Schichten sind nachfolgend aufgeführt. Das Implantat wird mit einer Strahlendosis (Co-60) von 25 kGy sterilisiert.

Die SiₓO_{y}C_{z}-Schichten wurden mit Hilfe einer Atmosphärendruckplasmaanlage (PFW10) der Firma Plasmatreat durchgeführt. Das Druckluftplasma brannte bei einer Transformator-Entladungsspannung von 300 V. In den relaxierenden Plasmastrahl wurde gasförmiges Hexamethydisiloxan (20 g/h) mit Hilfe des Trägergases Stickstoff (2 l/h) zugeführt. Bei einem Abstand von 6 mm zwischen Substrat und Beschichtungskopf ergab sich nach einem Behandlungszyklus (60 m/min) eine Schichtdicke von 40 nm.

Die Abscheidung der partikulären Silberschicht erfolgte mit der gleichen Atmosphärendruckanlage der Firma PlasmaTreat. In das relaxierende Druckluftplasma wurde ein Silberdraht mit einem Durchmeseeer von 1 mm gehalten. Nach einer Zyklenzahl von 20 bei einem Silberdrahtvorschub von 1µm/min ergibt sich eine Silberkonzentration von ca. 2 µg/cm².

### Beispiel 3:

Die Poren der strukturierten Implantate aus Beispiel 1 und 2 werden als Beispiel mit dem Antibiotikum (AB) Gentamicin gefüllt, indem die Implantate für eine halbe Stunde in eine wässrige Gentamicinlösung mit einer Konzentration von 10 µg/ml getaucht werden.

Die antimikrobielle Wirksamkeit wird mit Hilfe von Bakterien (E. coli) getestet. Hierzu wird das mit dem AB getränkte Implantat zusammen mit den Bakterien inkubiert. Vorzugsweise so, dass eine Suspension (100 µL) mit Bakterien (OD 600 nm of 1.0 ≈ 5 x 108 cells/mL) auf das Implantat pipettiert wird. Implantat (mit und ohne zugesetztes AB) und Bakterien werden mit einem Deckglas bedeckt, um Austrocknungseffekte zu vermeiden, und für mehrere Stunden bei 37 °C im Brutschrank inkubiert. Nach dieser Zeit werden die Bakteriensuspensionen abgenommen, Verdünnungsreihen in Kulturmedium hergestellt und jeweils 50 µL auf Agarplatten ausplattiert. Parallel dazu können weitere 50 µL der Proben, versetzt mit 250 µL Kulturmedium, in die Vertiefungen einer Petrischale gegeben werden und mit Hilfe eines Multimode-Readers (Serie Mithras LB940 der Firma Berthold Technologies) das Wachstum bei 37 °C über 24 h in Echtzeit über die Veränderung der optischen Dichte (OD) verfolgt werden.

Die Daten zeigen, dass die neuartige poröse und mit einer Freisetzungskontrolle versehene Implantatbeschichtung das Bakterienwachstum komplett verhindert.

### Beispiel 4:

Die Poren der strukturierten Implantate aus Beispiel 1 und 2 werden mit dem Antibiotikum (AB) Gentamicin gefüllt, indem die Implantate für eine halbe Stunde in eine wässrige Gentamicinlösung mit einer Konzentration von 10 µg/ml getaucht werden.

Die so hergestellten Implantate werden in Anlehnung an die DIN EN ISO 10993-5 mit höheren Zellen der Linie L929 inkubiert. Dieses ermöglicht, eine Aussage über das zytotoxische Potential der Implantate zu erhalten. Hierfür werden die Implantate bei 37 °C und 5% CO₂ mit jeweils 10.000 L929 Zellen für 24 h inkubiert und die Vitalität der Zellenanalysiert. Die Daten zeigen, dass die Beschichtung sowohl mit als auch ohne eine Beladung mit Antibiotika und im Vergleich mit einer unbehandelten Titanoberfläche als völlig biokompatibel zu bewerten ist.

### Beispiel 5: Ausblutversuche mit Methylenblau als Modell für einen Wirkstoff / Einfluss der Abgabenkontrollschicht auf das Abgabeverhalten

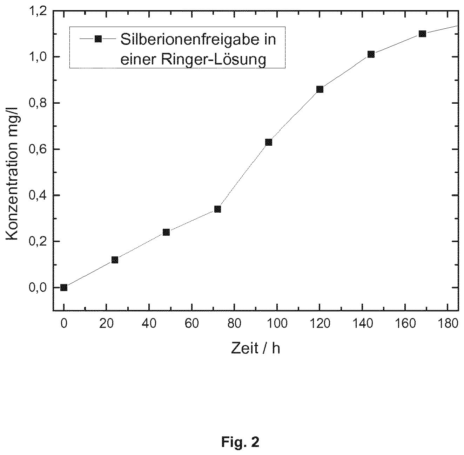

Um den Release des Wirkstoffs zu steuern, muss die Porengröße der oxidierten Oberfläche verjüngt werden, da die ca. 3 µm großen Poren den Wirkstoff sehr schnell wieder freigeben würden. Dies geschieht mit Hilfe einer nachträglichen Beschichtung aus der Gasphase, die Silber enthalten kann. Durch diese Verjüngung wird die zeitliche Freisetzung deutlich verzögert, ohne dass das Porenvolumen abnimmt, da sich die Beschichtung nicht in der Pore abscheidet. Das Silber liegt in Form von ca. 15 nm großen Partikeln vor, die einer SiO₂- oder TiO₂-Matrix fest eingebettet sind. Somit ist sichergestellt, dass nur die antibakteriell wirksamen Silberionen freigesetzt werden können und keine Partikel (vergl. Fig. 2).

### Versuchsbedingungen zur Herstellung der Wirkstoffträgerschicht (MAO-Schicht)

Die Herstellung der Titanproben (2 x 2 cm²) erfolge mit dem MAO-Verfahren. Als Elektrolyt wurde eine Kalziumazetatlösung mit einer Konzentration von 0,2 mol/l verwendet. Die Oxidation fand bei einer Spannung von 300 V statt. Die Schichtdicke der MAO-Schicht beträgt 5 ± 1 µm. Die Größenverteilung der Poren ist bimodal, d.h. der mittlere Durchmesser der großen Poren beträgt 2,52 µm und der mittlere Durchmesser der kleinen Poren beträgt 0,8 µm.

### Versuchsbedingung zur Herstellung der Abgabekontrollschicht

Die Abgabekontrollschicht wurde mit Hilfe der Plasmapolymerisation hergestellt. In einen Hochfrequenz-Plasmareaktor, der ein Volumen von 125 I besitzt, wurden Hexamethyldisiloxan und Sauerstoff mit einem Gasfluss von 100 sccm bzw. 5 sccm eingelassen. Die oxidierten Titanproben befanden sich auf der Plasmaentladungselektrode, so dass sie unter dem Einfluss der 450 V hohen Self-Bias-Spannung standen, die bei einer eingekoppelten Plasmaleistung von 100 W entsteht. Der Prozessdruck während der Abscheidung betrug 0,01 mbar. Nach der Abscheidedauer von 7 min ergab sich eine SiOₓ-Schichtdicke von 250 nm. Der Porendurchmesser wurde hierbei von 0,6 µm auf 0,1 µm (kleine Poren) bzw. von 1,52 auf 1 µm (große Poren) verjüngt.

### Messung der Freisetzung von z.B. Methylenblau

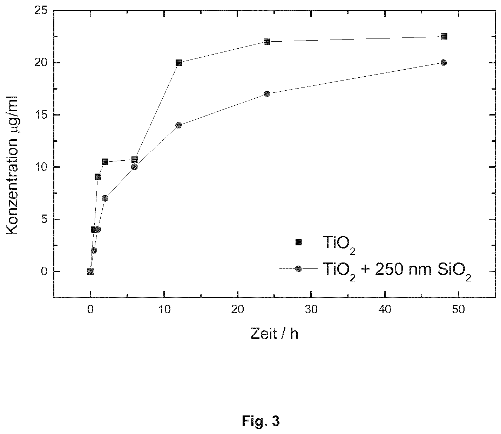

Die Imprägnierung (Beladung der Wirkstoffträgerschicht mit Wirkstoff) der Implantatoberfläche kann strategisch erst direkt vor / bei der Operation erfolgen. Zum Nachweis der Freisetzungsrate wurden die Chemotherapeutika Methylenblau (Fig. 3) und Gentamicin verwendet. Ohne die zusätzliche Verjüngungsschicht wird das Methylenblau in zwei Schüben freigegeben, da sich große und kleine Poren in der strukturierten Schicht befinden.

Die mit MAO oxidierten Titanproben wurden in einer konzentrierten Methylenblaulösung (50 g/l) über einen Zeitraum von 48 h imprägniert. Die Freisetzung des Methylenblaus wurde in destilliertem Wasser bei Raumtemperatur durchgeführt. Über die Messung der optischen Absorption kann die Konzentration des Methylenblaus in der Lösung bei verschiedenen Zeitpunkten bestimmt werden.

Innerhalb von zwei Stunden werden die großen Poren entleert und innerhalb von 24 Stunden die kleinen Poren. Die SiO₂- oder TiO₂-Schicht verjüngt den Durchmesser aller Poren, so dass die Freisetzungsrate geringer und ein langanhaltende Freisetzung erreicht wird.

### Beispiel 6: Bestimmung der Porengröße mit/ohne Verjüngungsschicht

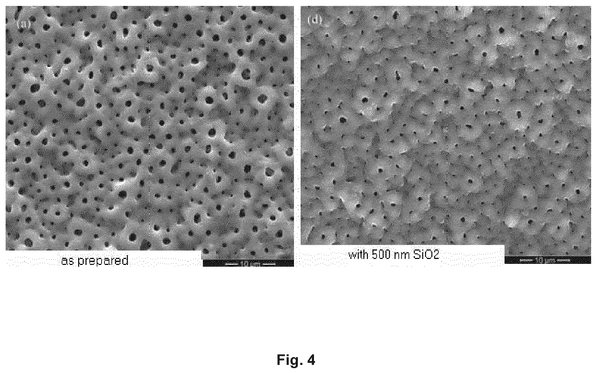

Zur Bestimmung der Porenöffnungsdurchmesser wurden vor und nach der Applikation der Verjüngungsschicht rasterelektronenmikroskopische (REM) Aufnahmen gemacht. Das linke REM-Bild aus Fig. 4 zeigt die Oberfläche der MAO-Schicht. Der mittlere Durchmesser der großen Poren beträgt ca. 2,5 µm und der mittlere Durchmesser der kleinen Poren ca. 0,8 µm. Das rechte REM-Bild aus Fig. 4 zeigt die Oberfläche nach der Beschichtung mit der Verjüngungsschicht. Der Durchmesser der großen Poren beträgt ca. 1,2 µm und der Durchmesser der kleinen Poren ist kleiner als 100 nm.

## Patentansprüche

1. Implantatkörper, umfassend ein Substrat (3) und auf dessen Oberfläche ein Schichtsystem aus einer Wirkstoffträgerschicht (2) und darauf angeordnet eine Abgabekontrollschicht (1), wobei
die Wirkstoffträgerschicht eine Vielzahl von offenen Poren (4) umfasst und
die Abgabekontrollschicht (1) eine aus der Gasphase abgeschiedene Schicht ist, die die Fläche der Porenöffnung der Poren (4) der Wirkstoffträgerschicht (2) reduziert, ohne sie zu schließen.

2. Implantatkörper nach Anspruch 1, wobei die Wirkstoffträgerschicht (2) eine metallhaltige oder metalloxidhaltige Schicht ist oder aus Metall oder Metalloxid besteht und es sich um Metalle oder Metalloxide der Elemente Ti, Fe, Cr, Co, V und/oder Ta handelt.

3. Implantatkörper nach Anspruch 1 oder 2, wobei die Abgabekontrollschicht (1) eine CVD-, PE-CVD- oder PVD-Schicht ist.

4. Implantatkörper nach einem der vorangehenden Ansprüche, wobei die Poren (4) in der Wirkstoffträgerschicht (2) mittels eines Verfahrens erzeugt wurden, ausgewählt aus der Gruppe bestehend aus Laserstrukturierung, Laser-PVD, Laser-CVD, Micro-Arc Oxidation, elektrochemische Oxidation und Anodisierung.

5. Implantatkörper nach einem der vorangehenden Ansprüche, wobei die Poren (4) wenigstens teilweise mit einem medizinischen Wirkstoff, bevorzugt einem antimikrobiellen Wirkstoff oder einem Chemotherapeutikum und besonders bevorzugt einem Antibiotikum gefüllt sind.

6. Implantatkörper nach einem der vorangehenden Ansprüche, wobei der Porendurchmesser 0,005 µm bis 2,0 µm, bevorzugt 0,1 µm bis 1,5 µm, besonders bevorzugt 0,5 µm gemessen mittels Rasterelektronenmikroskopie beträgt.

7. Implantatkörper nach einem der vorangehenden Ansprüche, wobei der Porenöffnungsdurchmesser 0,002 µm bis 1,0 µm nach Aufbringen der Abgabekontrollschicht, bevorzugt 0,05 µm bis 0,5 µm, besonders bevorzugt 0,1 µm gemessen mittels Rasterelektronenmikroskopie beträgt und der Porenöffnungsdurchmesser geringer ist als der Porendurchmesser.

8. Implantatkörper nach einem der vorangehenden Ansprüche, wobei die Abgabekontrollschicht (1) und/oder die Wirkstoffträgerschicht (2) Silber und/oder Kupfer umfasst.

9. Implantatkörper nach einem der vorangehenden Ansprüche, wobei die Abgabekontrollschicht (1) zu ≥ 90% aus Ti, C, O und/oder Si besteht, bezogen auf die in der Schicht enthaltenen Atome ohne H gemessen mittels XPS.

10. Verwendung einer Abgabekontrollschicht (1), wie in einem der vorangehenden Ansprüche definiert zur Verringerung des Porenöffnungsdurchmessers der Poren (4) auf einem Implantatkörper, wobei die Pore (4) nicht verschlossen wird und bevorzugt mit einem medizinischen Wirkstoff wenigstens teilweise befüllt ist.

11. Verfahren zum Erzeugen eines Implantatkörpers nach einem der Ansprüche 1 bis 9, umfassend die Schritte,
a) Bereitstellen eines Substrates (3) für den Implantatkörper,
b) Erzeugen einer porenhaltigen Wirkstoffträgerschicht (2) auf der Oberfläche des Implantatkörpers und
c) Abscheiden einer Abgabekontrollschicht (1) aus der Gasphase auf die Wirkstoffträgerschicht, so dass der Porendurchmesser der Poren (4) der Wirkstoffträgerschicht (2) verringert wird, ohne dass die Poren (4) verschlossen werden.

## Claims

1. Implant body comprising a substrate (3) and, on the surface thereof, a layer system composed of an active-ingredient carrier layer (2) and, arranged on top thereof, a release control layer (1),
the active-ingredient carrier layer comprising a multiplicity of open pores (4) and
the release control layer (1) being a layer which was deposited from the gaseous phase and which reduces the surface area of the pore opening of the pores (4) of the active-ingredient carrier layer (2) without closure thereof.

2. Implant body according to Claim 1, wherein the active-ingredient carrier layer (2) is a metal-containing or metal oxide-containing layer or consists of metal or metal oxide, and metals or metal oxides of the elements Ti, Fe, Cr, Co, V and/or Ta are concerned.

3. Implant body according to Claim 1 or 2, wherein the release control layer (1) is a CVD, PE-CVD or PVD layer.

4. Implant body according to any of the preceding claims, wherein the pores (4) in the active-ingredient carrier layer (2) were generated by means of a method selected from the group consisting of laser structuring, laser PVD, laser CVD, micro-arc oxidation, electrochemical oxidation and anodization.

5. Implant body according to any of the preceding claims, wherein the pores (4) are filled at least in part with an active medical ingredient, preferably an active antimicrobial ingredient or a chemotherapeutic and particularly preferably an antibiotic.

6. Implant body according to any of the preceding claims, wherein the pore diameter is from 0.005 µm to 2.0 µm, preferably 0.1 µm to 1.5 µm and particularly preferably 0.5 µm, as measured by means of scanning electron microscopy.

7. Implant body according to any of the preceding claims, wherein the pore opening diameter is from 0.002 µm to 1.0 µm after application of the release control layer, preferably 0.05 µm to 0.5 µm and particularly preferably 0.1 µm, as measured by means of scanning electron microscopy, and the pore opening diameter is smaller than the pore diameter.

8. Implant body according to any of the preceding claims, wherein the release control layer (1) and/or the active-ingredient carrier layer (2) comprises silver and/or copper.

9. Implant body according to any of the preceding claims, wherein the release control layer (1) consists of Ti, C, O and/or Si to an extent of ≥ 90%, based on the atoms which are present in the layer and which do not include H, as measured by means of XPS.

10. Use of a release control layer (1) as defined in any of the preceding claims for reducing the pore opening diameter of the pores (4) on an implant body, wherein the pore (4) is not closed and is preferably filled at least in part with an active medical ingredient.

11. Method for generating an implant body according to any of Claims 1 to 9, comprising the steps of
a) providing a substrate (3) for the implant body,
b) generating a pore-containing active-ingredient carrier layer (2) on the surface of the implant body and
c) depositing a release control layer (1) from the gaseous phase onto the active-ingredient carrier layer, with the result that the pore diameter of the pores (4) of the active-ingredient carrier layer (2) is reduced without closure of the pores (4).

## Revendications

1. Corps d'implant, comprenant un substrat (3) et, sur sa surface, un système de couches composé d'une couche porteuse de principe actif (2) et, disposée dessus, une couche de contrôle de libération (1), dans lequel
la couche porteuse de principe actif comprend une pluralité de pores (4) ouverts et
la couche de contrôle de libération (1) est une couche séparée de la phase gazeuse, qui réduit la surface de l'ouverture de pore des pores (4) de la couche porteuse de principe actif (2) sans la fermer.

2. Corps d'implant selon la revendication 1, dans lequel la couche porteuse de principe actif (2) est une couche contenant un métal ou contenant un oxyde de métal ou est constituée de métal ou d'oxyde de métal, et les métaux ou les oxydes de métal sont des éléments Ti, Fe, Cr, Co, V et/ou Ta.

3. Corps d'implant selon la revendication 1 ou 2, dans lequel la couche de contrôle de libération (1) est une couche de CVD (dépôt chimique en phase vapeur), de PE-CVD (dépôt chimique en phase vapeur activé par plasma) ou de PVD (dépôt physique en phase vapeur).

4. Corps d'implant selon l'une quelconque des revendications précédentes, dans lequel les pores (4) ont été produits dans la couche porteuse de principe actif (2) au moyen d'un procédé, choisi parmi le groupe constitué d'une structuration au laser, d'un procédé de PVD au laser, d'un procédé de CVD au laser, d'une oxydation micro arc, d'une oxydation électrochimique ou d'une anodisation.

5. Corps d'implant selon l'une quelconque des revendications précédentes, dans lequel les pores (4) sont remplis au moins en partie d'un principe actif médical, de manière préférée d'un principe actif antimicrobien ou d'un agent chimiothérapeutique et de manière particulièrement préférée d'un antibiotique.

6. Corps d'implant selon l'une quelconque des revendications précédentes, dans lequel le diamètre de pore est de 0,005 µm à 2,0 µm, de manière préférée de 0,1 µm à 1,5 µm, de manière particulièrement préférée de 0,5 µm selon une mesure au moyen d'une microscopie électronique à balayage.

7. Corps d'implant selon l'une quelconque des revendications précédentes, dans lequel le diamètre d'ouverture de pore est de 0,002 µm à 1,0 µm après l'application de la couche de contrôle de libération, de manière préférée de 0,05 µm à 0,5 µm, de manière particulièrement préférée de 0,1 µm selon une mesure au moyen d'une microscopie électronique à balayage et le diamètre d'ouverture de porte est inférieur au diamètre de pore.

8. Corps d'implant selon l'une quelconque des revendications précédentes, dans lequel la couche de contrôle de libération (1) et/ou la couche porteuse de principe actif (2) comprennent de l'argent et/ou du cuivre.

9. Corps d'implant selon l'une quelconque des revendications précédentes, dans lequel la couche de contrôle de libération (1) est constituée à ≥ 90 % de Ti, C, O et/ou Si, par rapport aux atomes contenus dans la couche sans H selon une mesure au moyen d'une spectroscopie XPS.

10. Utilisation d'une couche de contrôle de libération (1), telle que définie dans l'une quelconque des revendications précédentes, pour réduire le diamètre d'ouverture de pore des pores (4) sur un corps d'implant, dans laquelle le pore (4) n'est pas fermé et est rempli au moins en partie de manière préférée d'un principe actif médical.

11. Procédé pour produire un corps d'implant selon l'une quelconque des revendications 1 à 9, comprenant les étapes :
a) de fourniture d'un substrat (3) pour le corps d'implant,
b) de production d'une couche porteuse de principe actif (2) contenant des pores sur la surface du corps d'implant, et
c) de séparation d'une couche de contrôle de libération (1) de la phase gazeuse sur la couche porteuse de principe actif de sorte que le diamètre de pore des pores (4) de la couche porteuse de principe actif (2) est réduit sans que les pores (4) ne soient fermés.
